# EUROPEAN PATENT APPLICATION

(11) **EP 4 600 373 A1**
(43) Date of publication of application: **13.08.2025**
(21) Application number: 23874953.5
(22) Date of filing: 06.10.2023
(51) Int. Cl.: C12Q 1/68, C12N 15/12, C12Q 1/6883

(54) **DATA COLLECTION METHOD AND KIT FOR DETERMINING LIKELIHOOD OF DEVELOPING ALZHEIMER'S DISEASE**

(30) Priority: 06.10.2022 JP 2022161607
(71) Applicant: Kobayashi, Nobuyuki, Tokyo 113-8510 (JP)
(72) Inventor: Kobayashi, Nobuyuki, Tokyo 113-8510 (JP)
(74) Representative: Nederlandsch Octrooibureau
(86) International application number: PCT/JP2023/036475
(87) International publication number: WO 2024/075828

(57) **Abstract**

A data collection method for determining the risk of developing Alzheimer's disease in subjects from biological samples, that includes measurement of DNA methylation levels in a CpG island containing a cytosine residue selected from a group comprising cytosine residues at positions 24, 56, 75, 81, 98, 103, 115, 126, 134, 142, 153 and 179 in the nucleotide sequence of the COASY gene promoter region indicated as SEQ ID NO:1, wherein a higher level of methylation as compared to NCs indicates a greater likelihood of developing Alzheimer's disease.

## Description

### TECHNICAL FIELD

This invention concerns a data collection method and kit for determining the likelihood of developing Alzheimer's disease.

Priority is claimed on Japanese Patent Application No. 2022-161607 filed on October 6, 2022, the content of which is incorporated herein by reference.

### BACKGROUND ART

Alzheimer's disease (AD) is the most common form of dementia. AD is considered to be due to deposition of amyloid β and phosphorylated tau in brain tissue, but its pathogenesis mechanism remains unknown. Since genetic factors, aging and environmental factors, such as lifestyle habits and infections, are involved in epigenetics, these factors are thought to be involved in the pathogenesis of AD.

Diagnosis for early discovery of AD is made by medical specialists but accurate diagnosis is difficult and can only be made when the disease has developed. Testing for Amyloid β in spinal fluid and positron emission tomography (PET) are commonly used examination methods. However, the former is highly invasive, PET is costly, only a limited number of institutions can conduct these examinations, and they are not covered by health insurance, making them impractical clinically.

No blood biomarker for the diagnosis of AD is currently available in clinical practice. The present inventor comprehensively quantified changes in methylation levels in blood DNA in healthy individuals (NCs), patients with AD and patients with amnestic mild cognitive impairment (aMCI), an earlier stage of AD, and discovered that DNA methylation was significantly reduced in the NCAPH2 (LMF2) gene promoter region in patients with AD and aMCI, and also that DNA methylation was significantly elevated in the COASY and SPINT1 gene promoter regions. The inventor previously reported that these biomarkers could potentially be useful in the early diagnosis of dementia and differentiation of dementia types (Non Patent Documents 1-3).

### Citation List

### Non Patent Documents

Non Patent Document 1: Kobayashi N, Shinagawa S, Nagata T, Shimada K, Shibata N, Ohnuma T, Kasanuki K, Arai H, Yamada H, Nakayama K, Kondo K. Development of Biomarkers Based on DNA Methylation in the NCAPH2/LMF2 Promoter Region for Diagnosis of Alzheimer's Disease and Amnesic Mild Cognitive Impairment. PLoS One 2016;11(1): e0146449.
Non Patent Document 2: Kobayashi N, Shinagawa S, Nagata T, Shimada K, Shibata N, Ohnuma T, Kasanuki K, Arai H, Yamada H, Nakayama K, Kondo K. Usefulness of DNA Methylation Levels in COASY and SPINT1 Gene Promoter Regions as Biomarkers in Diagnosis of Alzheimer's Disease and Amnestic Mild Cognitive Impairment. PLoS One 2016;11(12): e0168816.
Non Patent Document 3: Kobayashi N, Shinagawa S, Niimura H, Kida H, Nagata T, Tagai K, Shimada K, Oka N, Shikimoto R, Noda Y, Nakajima S, Mimura M, Shigeta M, Kondo K. Increased blood COASY DNA methylation levels a potential biomarker for early pathology of Alzheimer's disease. Sci Rep 2020;10(1): 12217.

### SUMMARY OF INVENTION

### Technical Problem

The COASY gene promoter region has multiple CpG islands but it is not known in which of them DNA methylation is associated with AD. In perfecting the invention, the inventor found that the it was useful for determining the likelihood of developing AD from methylation levels in specific CpG islands in the COASY gene promoter region. Thus, the purpose of the present invention is to provide a useful technique for determining the likelihood of subjects developing AD by measuring DNA methylation levels in specific CpG islands in the COASY gene promoter region.

### Solution to Problem

The present invention includes the following aspects.
[1] The present invention concerns a data collection method for determining the likelihood of developing AD in subjects from biological samples, that includes measurement of DNA methylation levels in a CpG island containing a cytosine residue selected from a group comprising cytosine residues at positions 24, 56, 75, 81, 98, 103, 115, 126, 134, 142, 153 and 179 in the nucleotide sequence of the COASY gene promoter region indicated as SEQ ID NO:1, wherein a higher level of methylation compared to normal controls (NCs) indicates a greater likelihood of developing AD.
[2] Data collection method described in [1] wherein biological samples are blood.
[3] Kit for determining likelihood that subjects will develop AD that includes primers or probes for amplification of DNA in a CpG island containing a cytosine residue selected from a group consisting of cytosine residues at positions 24, 56, 75, 81, 98, 103, 115, 126, 134, 142, 153 and 179 in the nucleotide sequence of the COASY gene promoter region indicated as SEQ ID NO:1.
[4] Kit stated in [3], wherein the primer for amplifying DNA in the CpG island containing cytosine residue at position 24 in the nucleotide sequence of the COASY gene promoter region indicated as SEQ ID NO:1 is a primer set comprising forward primer consisting of the nucleotide sequence indicated as SEQ ID NO:2 and a reverse primer consisting of the nucleotide sequence indicated as SEQ ID NO:9.

### Advantageous Effects of Invention

The present invention provides a useful technique for determining the likelihood that a subject will develop AD.

### BRIEF DESCRIPTION OF DRAWINGS

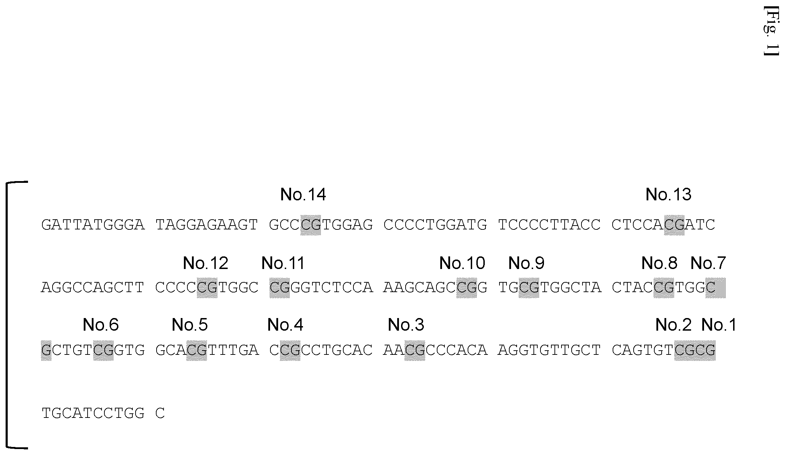

[Fig. 1] Shows the sequence of nucleotides in the COASY gene promoter region indicated as SEQ ID NO: 1 and the locations of CpG islands Nos. 1-14. Shaded DNA areas (CGs) indicate the locations of CpG islands.

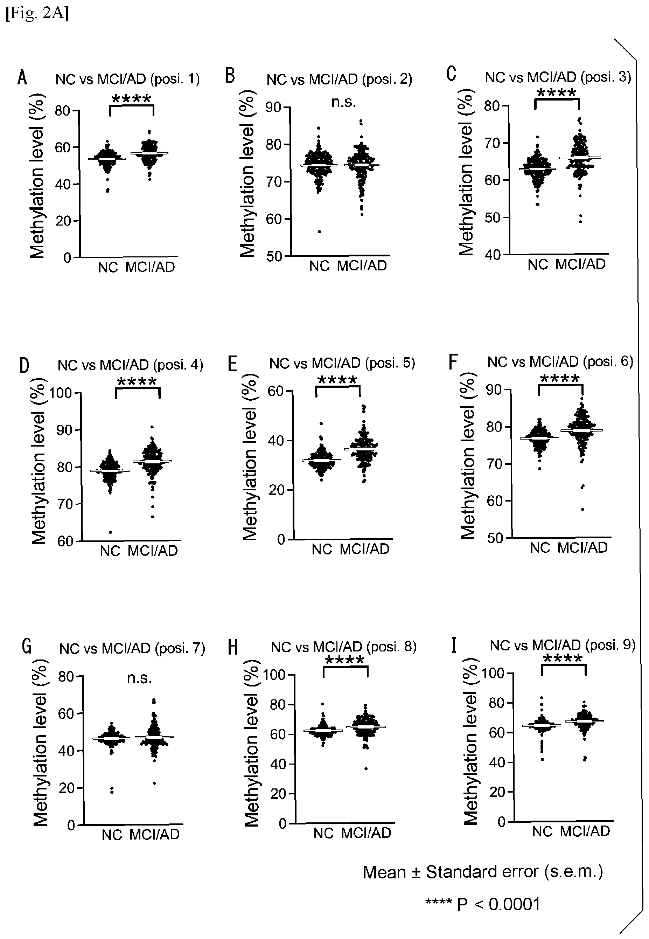

[Fig. 2A] Shows graphs for DNA methylation levels in CpG islands Nos. 1-9 of the COASY gene promoter region for non-dementia elderly individuals (NCs) and patients with AD and aMCI together. Panels A-I show DNA methylation levels for CpG islands Nos. 1-9, respectively.

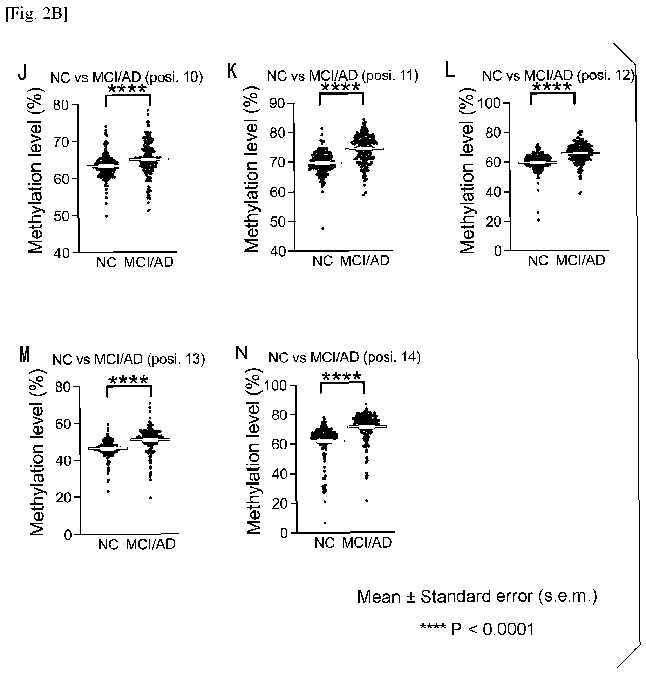

[Fig. 2B] Shows graphs for DNA methylation levels in CpG islands Nos. 10-14 of the COASY gene promoter region for NCs and patients with AD and aMCI together. Panels J-N show DNA methylation levels for CpG islands Nos. 10-14, respectively.

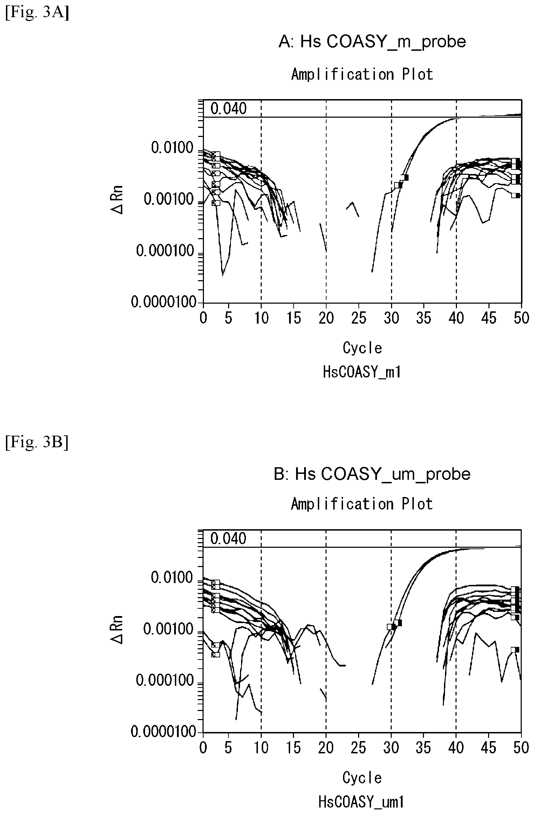

[Fig. 3A] Shows amplification plots for TacMan real-time PCR with the bisulfited DNA of CpG island No. 14 in the COASY gene promoter region as a template for DNA methylation rates of 100%, 75%, 50%, 25%, 5% and 0%, using a forward primer consisting of the nucleotide sequence indicated as SEQ ID NO:2, a reverse primer consisting of the nucleotide sequence indicated as SEQ ID NO:3 and a methylated DNA detection probe consisting of the nucleotide sequence indicated as SEQ ID NO:4.
[Fig. 3B] Shows amplification plots for TacMan real-time PCR with the bisulfited DNA of CpG island No. 14 in the COASY gene promoter region as a template for DNA methylation rates of 100%, 75%, 50%, 25%, 5% and 0%, using a forward primer consisting of the nucleotide sequence indicated as SEQ ID NO:2, a reverse primer consisting of the nucleotide sequence indicated as SEQ ID NO:3 and an unmethylated DNA detection probe consisting of the nucleotide sequence indicated as SEQ ID NO:5.

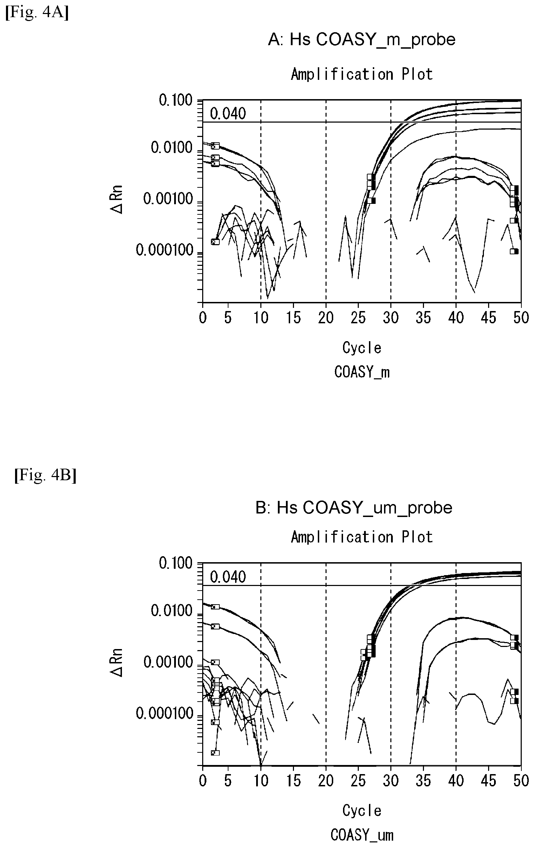

[Fig. 4A] Shows amplification plots for TacMan real-time PCR with the bisulfited DNA of CpG island No. 14 in the COASY gene promoter region as a template for DNA methylation rates of 100%, 75%, 50%, 25%, 5% and 0%, using a forward primer with the thymine residues at positions 3 and 20 in the nucleotide sequence indicated as SEQ ID NO:2 as Locked Nucleic Acids (LNAs), a reverse primer with the adenine residues at positions 8 and 20 in the nucleotide sequence indicated as SEQ ID NO:9 as LNAs and a methylated DNA detection probe consisting of the nucleotide sequence indicated as SEQ ID NO:4.
[Fig. 4B] Shows amplification plots for TacMan real-time PCR with the bisulfited DNA of CpG island No. 14 in the COASY gene promoter region as a template for DNA methylation rates of 100%, 75%, 50%, 25%, 5% and 0%, using a forward primer with the thymine residues at positions 3 and 20 in the nucleotide sequence indicated as SEQ ID NO:2 as Locked Nucleic Acids (LNAs), a reverse primer with the adenine residues at positions 8 and 20 in the nucleotide sequence indicated as SEQ ID NO:9 as LNAs and an unmethylated DNA detection probe consisting of the nucleotide sequence indicated as SEQ ID NO:5.

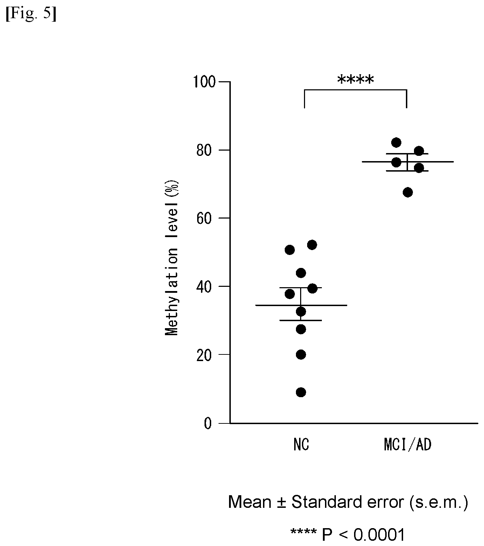

[Fig. 5] Graph showing methylation levels in CpG island No. 14 in the COASY gene promoter region for blood samples from NCs and AD patients and aMCI patients together using a forward primer with the thymine residues at positions 3 and 20 in the nucleotide sequence indicated as SEQ ID NO:2 as LNAs, a reverse primer with the adenine residues at positions 8 and 20 in the nucleotide sequence indicated as SEQ ID NO:9 as LNAs, as well as a probe for detecting methylated DNA consisting of the nucleotide sequence indicated as SEQ ID NO:4 or a probe for detecting unmethylated DNA consisting of the nucleotide sequence indicated as SEQ ID NO:5.

### DESCRIPTION OF EMBODIMENTS

### [Data collection method]

In one embodiment, the invention provides a data collection method for determining the likelihood that a subject will develop AD that includes measurement of DNA methylation levels in a CpG island containing a cytosine residue selected from a group of cytosine residues at positions 24, 56, 75, 81, 98, 103, 115, 126, 134, 142, 153 and 179 in a nucleotide sequence of the COASY gene (NM_001042529) promoter region indicated as SEQ ID NO:1, wherein higher methylation levels compared with NCs indicates that a subject is at risk of developing AD.

As described later in the working examples, the method in the present embodiment allows data to be collected for the purpose of determining the likelihood that a subject will develop AD. However, the data collection method does not include a process for a doctor to make such a determination. The method of this embodiment may also be described as a procedure for determining whether there is a likelihood of a subject developing AD.

In the method of the present embodiment, the DNA methylation level in a CpG island containing a cytosine residue selected from a group of cytosine residues at positions 24, 56, 75, 81, 98, 103, 115, 126, 134, 142, 153 and 179 in the nucleotide sequence of the COASY gene promoter region indicated as SEQ ID NO:1 is the data for determining whether a subject is at risk of developing AD.

Additionally, in the method of the present embodiment, the control is the level of DNA methylation in the aforementioned CpG island of the COASY gene promoter region in biological samples from subjects who are known not to have developed AD beforehand. Such subjects include healthy individuals and healthy elderly individuals.

The method of the present embodiment involves measuring the DNA methylation level in one or multiple CpG islands containing cytosine residues selected from a group of cytosine residues at positions 24, 56, 75, 81, 98, 103, 115, 126, 134, 142, 153 and 179 in the nucleotide sequence of the COASY gene promoter region indicated as SEQ ID NO:1 in biological samples from subjects, and allows it to be determined that a subject is at risk of developing AD if methylation levels in cytosine residues are higher as compared to NCs.

In the present embodiment, the CpG islands that contain cytosine residues at positions 24, 56, 75, 81, 98, 103, 115, 120, 126, 134, 142, 153, 177 or 179 in the nucleotide sequence of the COASY gene promoter region indicated as SEQ ID NO:1 are respectively referred to as CpG islands 14-1. Figure 1 shows the nucleotide sequence in the COASY gene promoter region indicated as SEQ ID NO: 1 and the positions of CpG islands 1-14 in this nucleotide sequence. Table 1 shows the relationship between cytosine residue locations in SEQ ID NO:1 and CpG island numbers.

**[Table 1]**

| CpG island No. | Positions of cytosine residues in SEQ ID NO:1 |
|---|---|
| 14 | 24 |
| 13 | 56 |
| 12 | 75 |
| 11 | 81 |
| 10 | 98 |
| 9 | 103 |
| 8 | 115 |
| 7 | 120 |
| 6 | 126 |
| 5 | 134 |
| 4 | 142 |
| 3 | 153 |
| 2 | 177 |
| 1 | 179 |

In the method of this embodiment, there are no particular restrictions on the biological samples collected as long as they provide DNA containing data for the purpose of determining the likelihood of developing AD in subjects. They could be blood, hair, saliva or skin, but from the aspects of low invasiveness and abundant DNA content, blood is preferable. Specifically, genome DNA present in biological samples is extracted and DNA methylation levels in the aforementioned CpG islands of the COASY gene promoter region are measured.

CpG island refers to a region where the frequency of CpG, a dinucleotide sequence where guanine follows cytosine, is higher compared to other regions in the genome. The "p" in CpG indicates a phosphodiester bond between cytosine and guanine. Many mammalian genes are known to contain CpG islands within or in the vicinity of promoters.

Among the CpG islands in the COASY promoter region used for the method of the current embodiment, the Cg numbers (probe ID numbers used in Illumina Infinitum HD Methylation Assay Kit (Illumina)) of CpG islands Nos. 7, 6, 5, 4 and 1 are CG15138339, CG19618279, CG05465955, CG25135457 and CG01756799, respectively.

A suitable method of detecting methylated cytosine residues is one using bisulfite treatment of DNA fragments. In bisulfite treatment, cytosine residues are deaminated and converted to uracil residues, but cytosine residues remain unchanged if methylated. A commercially available kit such as EZ DNA Methylation Kit (Zymo Research) may be used for bisulfite treatment.

After bisulfite treatment, methylation of cytosine residues may be detected by sequencing of DNA fragments. The percentage of methylated cytosine residues can be measured by determining the nucleotide sequences of all DNA fragments through sequencing using a next generation sequencer.

Alternatively, using DNA fragments after bisulfite treatment as templates, methylation of cytosine residues can be detected by means of a PCR method using primers that distinguish between cytosine residues and uracil residues. The percentage of methylated cytosine residues may be measured by performing quantitative PCR and creating a high-resolution melting curve as a function of reaction temperature for PCR products.

Another method is as follows. A probe capable of hybridization to the target region is designed. After bisulfite treatment, DNA fragments are subjected to whole genome amplification and then fragmented, both processes using enzymes, fragments are hybridized to beads to which a probe has been attached and fluorescently labeled nucleotides are incorporated by a single nucleotide extension reaction. The percentage of methylated cytosine residues can then be measured by measuring the fluorescence intensity using fluorescent dye-labeled antibodies. This procedure can be conducted using a commercially available kit; for instance, Illumina Infinitum HD Methylation Assay Kit (Illumina), with bead analysis by means of iScan (Illumina) to measure percentage methylation.

Yet another method of measuring the percentage of methylated cytosine residues is as follows. With DNA fragments following bisulfite treatment as templates, PCR amplification of target regions is carried out and then transcription into RNA. Next, using an RNase, nucleotide specific fragmentation is performed and methylated and unmethylated DNA fragments are detected from their differing molecular weights using a time-of-flight mass spectrometer. The percentage methylation may be measured using a commercially available analysis kit; for instance, Mass ARRAY Epi-TYPER (SEQUENOM).

One more way of doing this is a PCR method using bisulfited DNA fragments as templates and primers that distinguish between cytosine and uracil residues. By this method, the percentage methylation of cytosine residues is measured by means of quantitative PCR using probes labeled with 2 fluorescent dyes that allow cytosine and uracil to be distinguished.

### [Kit]

In 1 embodiment, this invention provides a kit to determine the likelihood of subjects developing AD, that includes primers or probes for the amplification of DNA methylation regions in CpG islands containing cytosine residues selected from a group of cytosine residues at positions 24, 56, 75, 81, 98, 103, 115, 126, 134, 142, 153 and 179 in the nucleotide sequence of the COASY gene promoter region indicated as SEQ ID NO:1.

The kit of this embodiment allows the percentage DNA methylation in CpG islands containing cytosine residues selected from a group of cytosine residues at positions 24, 56, 75, 81, 98, 103, 115, 126, 134, 142, 153 and 179 in the aforementioned nucleotide sequence of the COASY gene promoter region to be measured.

Examples of primers for amplifying DNA in the CpG island (CpG island No. 14) containing the cytosine residue at position 24 in the nucleotide sequence of the COASY gene promoter region indicated as SEQ ID NO:1 are those in the primer set shown in Table 2 below, comprising a forward primer consisting of the nucleotide sequence indicated as SEQ ID NO:2 and a reverse primer consisting of the nucleotide sequence indicated as SEQ ID NO:9. Primers may incorporate LNAs in whole or in part. Incorporation of LNAs in primers can increase the melting temperature (Tm) of the DNA double strand and enhance binding to the target. For instance, with the forward primer consisting of the nucleotide sequence indicated as SEQ ID NO:2, thymine residues at positions 3 and 20 should be LNAs. For the reverse primer consisting of the nucleotide sequence indicated as SEQ ID NO:9, adenine residues at positions 8 and 20 should be LNAs.

**[Table 2]**

| SEQ ID NO | Sequence |
|---|---|
| 2 | GATTATGGGATAGGAGAAGTG |
| 9 | CACCACAAACTACTCCTAAAC |

Examples of probes for amplifying DNA in the CpG island (CpG island No. 14) containing the cytosine residue at position 24 in the nucleotide sequence of the COASY gene promoter region indicated as SEQ ID NO:1 are a probe for detecting methylated DNA consisting of the nucleotide sequence indicated as SEQ ID NO:4 and a probe for detecting unmethylated DNA consisting of the base sequence indicated as SEQ ID NO:5, shown in Table 3 below.

**[Table 3]**

| SEQ ID NO | Sequence |
|---|---|
| 4 | TCGTGGAGTTTTTGGATGTTTTTTTATTTTTTA |
| 5 | TTGTGGAGTTTTTGGATGTTTTTTTATTTTTTA |

The kit of the present embodiment may also include bisulfite, besides the primers and probes mentioned above. It may also include various reagents and restriction enzymes such as for DNA extraction, hybridization and ligation, and PCR reactions.

### [Other embodiments]

In one other embodiment, the present invention may be used in the provision of treatment for AD. Through the measurement in patients' biological samples of DNA methylation levels in CpG islands containing cytosine residues selected from a group of cytosine residues at positions 24, 56, 75, 81, 98, 103, 115, 126, 134, 142, 153 and 179 in the nucleotide sequence of the COASY gene promoter region indicated as SEQ ID NO:1, treatment may be offered when the methylation levels in subjects are higher than in NCs.

As mentioned in the foregoing, the inventor has demonstrated that in the biological samples of subjects at risk of developing AD, DNA methylation levels in a CpG island containing a cytosine residue selected from a group of cytosine residues at positions 24, 56, 75, 81, 98, 103, 115, 126, 134, 142, 153 and 179 in the nucleotide sequence of the COASY gene promoter region indicated as SEQ ID NO:1 are significantly higher than in NCs. Therefore, when the cytosine residues are methylated, and the methylation level is significantly higher than in NCs, it can be concluded that such subjects are likely to develop AD.

In a treatment modality for this embodiment, the controls, biological samples and procedures for the measurement of DNA methylation in CpG islands would be the same as those mentioned in the foregoing.

Currently known treatments for AD include drug therapy, such as that with Aricept (registered trade mark) and Reminyl (registered trade mark), as well as non-drug therapy methods such as those targeting the patient's behavioral or psychological symptoms.

### Examples

The following examples illustrate the use of the present invention in more detail. However, its use is not limited to these specific examples.

### [Experimental Example 1]

Peripheral blood DNA from subjects was bisulfited. Then, with the bisulfited DNA as a template, the COASY gene promoter region indicated as SEQ ID NO: 1 was amplified by means of PCR using the pyrosequencing method. Primers used were a biotin-modified forward primer (5'-[Biotin] -GATTATGGGATAGGAGAAGTGTT-3') with the nucleotide sequence shown as SEQ ID NO:6 modified at the 5 side, a reverse primer consisting of the nucleotide sequence shown as SEQ ID NO:7 (5'-CCTAATCCAAAATCCCTCTTAC-3') and a sequence primer consisting of the nucleotide sequence shown as SEQ ID NO:8 (5'-ACCACAAACTACTCCTAA-3'). The PCR amplification products obtained were adsorbed onto beads and sequencing of the bisulfited COASY gene promoter region was performed from the 3' side of the nucleotide sequence using PyroMark Q24 Advanced (Qiagen). There are 14 CpG islands in the sequence. The first CpG island to appear was designated as CpG island No. 1 and the last one to appear as CpG island No. 14. Figure 1 shows the nucleotide sequence in the COASY gene promoter region and the locations of CpG islands Nos. 1-14.

Next, 0.5-1.0 µg of peripheral blood DNA from a total of 173 patients with aMCI or AD, and 200 NCs was bisulfited using EZ DNA Methylation Kit (Zymo Research), the bisulfited DNA was sequenced according to the pyrosequencing method and the DNA methylation levels in CpG islands Nos. 1-14 in the COASY gene promoter region were measured.

The results are shown in Fig. 2A and 2B. As shown, in the nucleotide sequence of the COASY gene promoter region, excepting CpG islands No. 2 and 7, methylation levels in CpG islands Nos. 1, 3-6 and 8-14 in AD and aMCI patients were significantly higher than in NCs (non-dementia elderly subjects). CpG islands Nos. Nos. 1, 3-6 and 8-14 contain cytosine residues at positions 24, 56, 75, 81, 98, 103, 115, 126, 134, 142, 153 and 179, respectively.

Next, Area under the Curve (AUC) values for ROC curves were calculated separately for AD and aMCI patients together and NCs, for CpG islands Nos. 1-14. The results are shown in Fig. 4. As shown, the value of the AUC for the methylation level in CpG island No. 14 was the highest.

**[Table 4]**

| CpG island No. | AUC value | Std. Error | 95% confidence interval | P value |
|---|---|---|---|---|
| 1 | 0.7008 | 0.02737 | 0.6472 to 0.7545 | <0.0001 |
| 2 | 0.5002 | 0.0303 | 0.4408 to 0.5596 | 0.995 |
| 3 | 0.7436 | 0.0262 | 0.6922 to 0.7949 | <0.0001 |
| 4 | 0.7538 | 0.02575 | 0.7033 to 0.8042 | <0.0001 |
| 5 | 0.7869 | 0.02456 | 0.7387 to 0.8350 | <0.0001 |
| 6 | 0.7223 | 0.02759 | 0.6682 to 0.7763 | <0.0001 |
| 7 | 0.5153 | 0.03197 | 0.4526 to 0.5779 | 0.6105 |
| 8 | 0.6912 | 0.02897 | 0.6344 to 0.7480 | <0.0001 |
| 9 | 0.7361 | 0.02718 | 0.6828 to 0.7894 | <0.0001 |
| 10 | 0.6495 | 0.02937 | 0.5919 to 0.7070 | <0.0001 |
| 11 | 0.7938 | 0.02433 | 0.7461 to 0.8415 | <0.0001 |
| 12 | 0.7783 | 0.02486 | 0.7296 to 0.8270 | <0.0001 |
| 13 | 0.786 | 0.02624 | 0.7346 to 0.8374 | <0.0001 |
| 14 | 0.8142 | 0.02421 | 0.7668 to 0.8617 | <0.0001 |

### [Experimental Example 2]

Next, a method of measuring DNA methylation levels in CpG island No.14 in the COASY gene promoter region was devised.

Using a primer set comprising a forward primer consisting of the nucleotide sequence shown as SEQ ID NO:2 (5'-GATTATGGGATAGGAGAAGTG-3') and a reverse primer consisting of the nucleotide sequence shown as SEQ ID NO:3 (5'-AACTACTCCTAAACCAAAATACAC-3') and a methylated DNA detection probe consisting of the nucleotide sequence shown as SEQ ID NO:4 (hereafter, referred to as Hs COASY _m_probe) or an unmethylated DNA probe consisting of the nucleotide sequence shown as base SEQ ID NO:5 (hereafter referred to a Hs COASY _um_probe probe), TacMan real-time PCR was performed with bisulfited DNA from CpG island No.14 in the COASY gene promoter region as a template for methylation rates of 100%, 75%, 50%, 25%, 5% and 0%. Figure 3A and 3B show amplification plots for the use of the Hs COASY _m_probe and Hs COASY _um_probe, respectively.

Figure 3A shows that, with the Hs COASY_m_probe for detecting methylated DNA, amplification was possible for a 100% methylation rate sample but not for other samples. Also, Fig. 3B shows that with the Hs COASY _um_probe for detecting unmethylated DNA, amplification was possible for a 0% methylated sample, but not for other samples. A suggested reason for this is poor amplification efficiency of the primer set used.

To address this, LNAs were incorporated in primers. A forward primer (hereafter, referred to as COASY_F_LNA) with thymine residues at positions 3 and 20 in the nucleotide sequence indicated as SEQ ID NO:2 as LNAs and a reverse primer (hereafter, referred to as COASY _R_LNA) with adenine residues at positions 8 and 20 in the nucleotide sequence indicated as SEQ ID NO:9 as LNAs were created and the melting temperature (Tm) of the double strand was raised. Figure 4A and 4B show amplification plots for the use of the Hs COASY_m_probe and Hs COASY _um_probe, respectively.

As shown in Fig. 4A, with the Hs COASY _m_probe for detecting methylated DNA, amplification was possible for 100%, 75% and 50% methylation rate samples. As shown in Fig. 4B, with the Hs COASY _um_probe for detecting unmethylated DNA, amplification was possible for 0%, 5%, 25% and 50% methylation rate samples. Digital PCR was not performed in this working example. If digital PCR had been performed, based on the above results, amplification should have been possible for all samples with the Hs COASY _m_probe and Hs COASY _um_probe since digital PCR would raise measurement sensitivity.

### [Experimental Example 3]

In a total of 5 patients with aMCI or AD, and 9 NCs, the DNA methylation rate in CpG island 14 in the COASY gene promoter region was measured using COASY_F_LNA as the forward primer, COASY_R_LNA as the reverse primer, the Hs COASY _m_probe for detecting methylated DNA and the Hs COASY _um_probe for detecting unmethylated DNA. Specifically, using bisulfited DNA with methylation rates of 100%, 75%, 50%, 25%, 5% and 0%, calibration curves were created. Then, using the Hs COASY_m_probe and Hs COASY_m_probe with each curve, the levels of methylated and unmethylated DNA were respectively quantified. Methylated and unmethylated DNA amounts were corrected so that their total was 100%.

The results are shown in Fig. 5. As shown, the methylated DNA levels in CpG island No. 14 for the AD patients and aMCI patients together were significantly higher compared to NCs (P<0.0001, Welch's t test).

### INDUSTRIAL APPLICABILITY

The present invention provides a technique for determining the likelihood of developing AD.

### SEQUENCE LISTING

PC37593_WA_Sequence_Listing.xml

## Claims

1. A data collection method for determining the risk of developing Alzheimer's disease in subjects from biological samples, that includes measurement of DNA methylation levels in a CpG island containing a cytosine residue selected from a group comprising cytosine residues at positions 24, 56, 75, 81, 98, 103, 115, 126, 134, 142, 153 and 179 in the nucleotide sequence of the COASY gene promoter region indicated as SEQ ID NO: 1, wherein a higher level of methylation as compared to NCs indicates a greater likelihood of developing Alzheimer's disease.

2. The data collection method according to Claim 1, wherein biological samples are blood.

3. A kit for determining the likelihood of subjects developing Alzheimer's disease that includes primers or probes for DNA amplification in a CpG island containing a cytosine residue selected from a group consisting of cytosine residues at positions 24, 56, 75, 81, 98, 103, 115, 126, 134, 142, 153 and 179 in a nucleotide sequence of the COASY gene promoter region indicated as SEQ ID NO: 1.

4. The kit according to Claim 3, wherein the primer for amplifying DNA in the CpG island containing a cytosine residue at position 24 in the nucleotide sequence of the COASY gene promoter region indicated as SEQ ID NO: 1 is a primer set comprising a forward primer consisting of the nucleotide sequence indicated as SEQ ID NO:2 and a reverse primer consisting of the nucleotide sequence indicated as SEQ ID NO:9.
